# EUROPEAN PATENT APPLICATION

(11) **EP 3 599 032 A1**
(43) Date of publication of application: **29.01.2020**
(21) Application number: 19188464.2
(22) Date of filing: 25.07.2019
(51) Int. Cl.: B05C 17/005

(54) **DISPENSING TUBE FOR DISPENSING LIQUID MATERIALS**

(30) Priority: 26.07.2018 US 201862703576 P; 24.07.2019 US 201916520871
(71) Applicant: Nordson Corporation, Westlake, OH 44145 (US)
(72) Inventor: MACINDOE, William, East Providence, RI Rhode Island 02914 (US); SPRINGHORN, Robert W, East Providence, RI Rhode Island 02914 (US)
(74) Representative: Findlay, Alice Rosemary

(57) **Abstract**

A tube for use with a dispensing syringe for dispensing a liquid material includes a body having a proximal end and a distal end opposite the proximal end. The proximal end is connected to a hub that is connected to the dispensing syringe. The tube also includes a channel extending through the body between the proximal end and the distal end, an inlet at the proximal end, and an outlet at the distal end. The inlet and the outlet are in fluid communication with the channel. The inlet receives the liquid material into the channel. The channel is partially tapered in the distal direction such that the proximal end has a first inner diameter and the outlet has a second inner diameter that is smaller than the first inner diameter.

## Description

The present disclosure relates generally to dispensing liquid materials, and more particularly to a tube attachment for syringes for dispensing liquid materials.

Various types of dispensers are used in many industries for applying liquids, such as adhesives, solder, and other similar materials, onto substrates during an assembly process. One type of liquid dispenser is a syringe-type dispenser having a dispenser body defining a barrel reservoir for holding a supply of liquid material to be dispensed. A dispensing tip is coupled to the syringe at one end, and is in fluid communication with the reservoir. A piston disposed in the reservoir is movable therein to pressurize the liquid in the reservoir and thereby dispense a small amount of liquid from the dispensing tip and onto a substrate.

Existing dispensers use dispensing tips that cause liquid flow at relatively large diameters, variable rates between applications, and with pauses in dispensing due to maintenance or replacement of parts. Existing dispensers also require high pressures to achieve desired flow rates at relatively small diameters.

According to an aspect of the disclosure, a tube for use with a dispensing syringe for dispensing a liquid material includes a body having a proximal end and a distal end opposite the proximal end. The proximal end is configured to be connected to a hub that is configured to be connected to the dispensing syringe. The tube also includes a channel extending through the body between the proximal end and the distal end, an inlet at the proximal end, and an outlet at the distal end. The inlet and the outlet are in fluid communication with the channel. The inlet is configured to receive the liquid material into the channel. The channel is partially tapered in the distal direction such that the proximal end has a first inner diameter and the outlet has a second inner diameter that is smaller than the first inner diameter.

According to another aspect, a dispensing apparatus for dispensing a liquid material includes a barrel having an interior compartment configured to receive the liquid material therein and a dispensing tip.. The dispensing tip is configured to be removably connected to the barrel and has a chamber in fluid communication with the interior compartment of the barrel. The chamber is configured to receive the liquid material from the interior compartment. The dispensing tip includes a dispensing tube that has a channel extending therethrough between in inlet at a proximal end and an outlet at a distal end. The dispensing tube is configured to receive the liquid material into the channel at the inlet and to dispense the liquid material out of the outlet. The channel is partially tapered in the distal direction.

Preferably the proximal end of the channel has a first inner diameter and the outlet of the channel has a second inner diameter that is smaller than the first inner diameter.

According to another aspect, a method of dispensing a liquid material includes providing the liquid material into a dispensing apparatus that has a tube for dispensing the liquid material and a channel extending through the tube, moving the liquid material through a first portion of the channel having a first inner diameter, moving the liquid material through a second, tapered portion of the channel to an outlet having a second inner diameter, and forming a deposit of the liquid material on a substrate. The second inner diameter is smaller than the first inner diameter.

The invention will now be further described by way of example with reference to the accompanying drawings, in which:
FIG. 1 illustrates an isometric view of a dispensing syringe with a dispensing tip including a dispensing tube;
FIG. 2 illustrates an isometric view of a dispensing tip with a dispensing tube;
FIG. 3 illustrates a cross-sectional view of a hub;
FIG. 4 illustrates an isometric view of a dispensing tube with adhesive;
FIG. 5 illustrates a cross-sectional view of the dispensing tube of FIG. 4;
FIG. 6 illustrates a cross-sectional view of another dispensing tube;
FIG. 7A illustrates a side perspective view of a dispensing tube;
FIG. 7B illustrates a cross-sectional view of the dispensing tube of FIG. 7;
FIG. 7C illustrates a cross-sectional view of a portion of the dispensing tube of FIGs. 7A and 7B; and
FIG. 7D illustrates a cross-sectional view of another dispensing tube.

Aspects of the disclosure will now be described in detail with reference to the drawings, wherein like reference numbers refer to like elements throughout, unless specified otherwise.

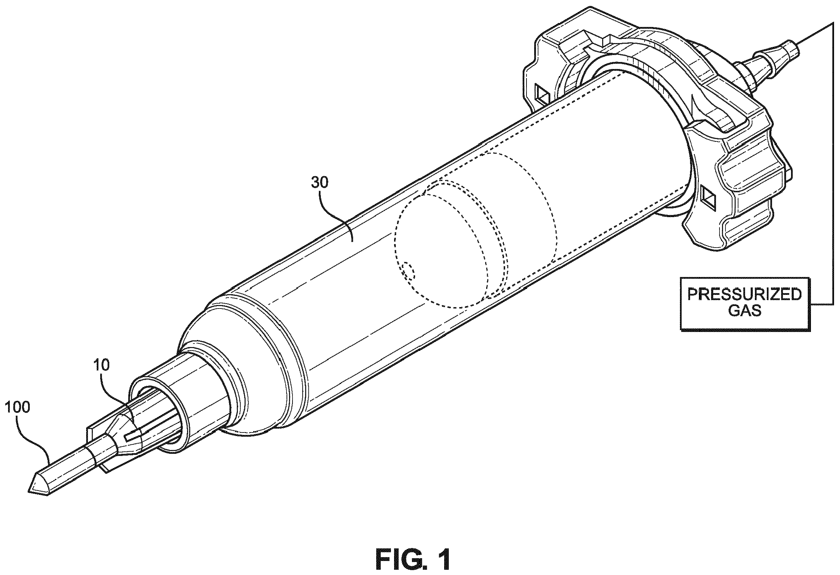

Liquid material may be dispensed in a variety of different ways and using different dispensers, dispensing mechanisms, and dispensing patterns. FIG. 1 illustrates an exemplary liquid dispensing syringe 30. The syringe 30 comprises an elongate tubular body having a dispensing end for dispensing liquid material therefrom. In some aspects, a piston may be slidably disposed within the syringe 30 and be slidably movable therein towards or away from the dispensing end. A dispensing tip 10 is removably coupled to the syringe 30 such that the material within the syringe 30 may be moved therefrom to the dispensing tip 10 and, subsequently, dispensed from a dispensing tube 100 connected, permanently or releasably, thereto. While a syringe 30 is depicted in the figures, it will be understood that any suitable container may be utilized to receive the liquid material and to engage with the dispensing tip 10.

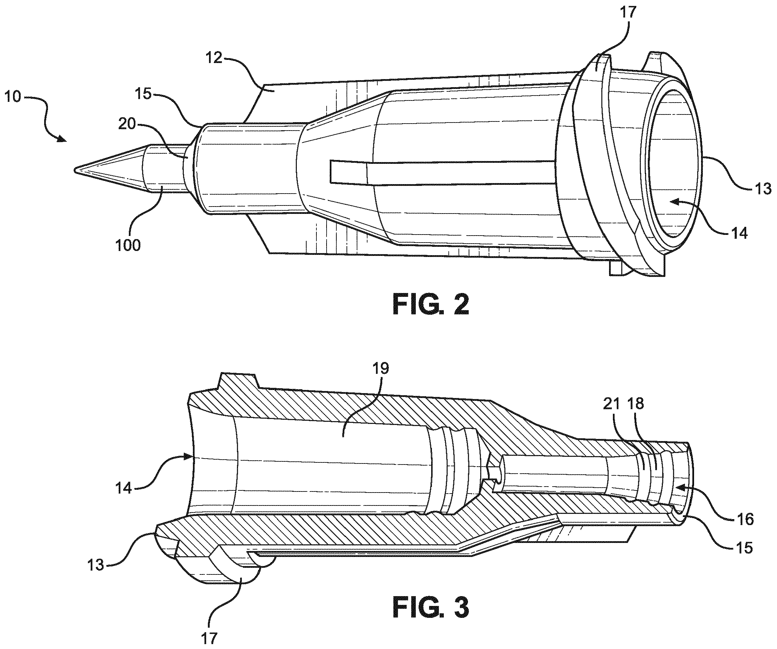

The dispensing tip 10 may engage with the syringe 30 or another suitable container via any known connection methods typically utilized in the field of dispensing, such as threads, Luer locks, snap fittings, friction fittings, or another retention mechanism. Referring to FIGs. 2 and 3, the dispensing tip 10 includes a hub 12 having a proximal end 13 and a distal end 15 opposite the proximal end 13. Threads 17 may be disposed at or adjacent to a proximal end 13 to help secure the dispensing tip 10 to the syringe 30.

A first opening 14 is disposed at the proximal end 13, and a second opening 16 is disposed at the distal end 15. The hub 12 is at least partially hollow inside and defines a chamber 19 that extends between the proximal end 13 and the distal end 15 and is in fluid communication with the first opening 14 and the second opening 16.

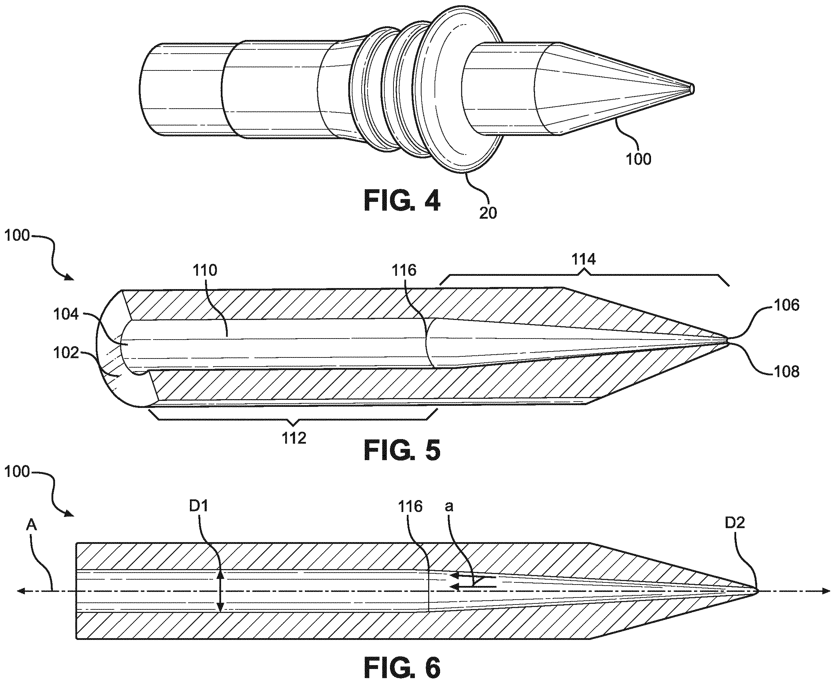

A dispensing tube 100 is coupled to the hub 12 of the dispensing tip 10 at the distal end 15. Referring to FIGs. 4-7D, the dispensing tube 100 is shown to have a substantially cylindrical portion and a substantially conical portion. It will be understood, however, that other shapes and arrangements of shapes are possible, such as prismatic, spherical or semi-spherical, trapezoidal, or another combination of shapes. The dispensing tube 100 has a proximal end 102 and a distal end 106 opposite the proximal end.

An inlet 104 is disposed at the proximal end 102 for receiving the liquid material that flows through the chamber 19 of the hub 12. The liquid material may pass from the chamber 19 through the second opening 16 and into the dispensing tube 100 through the inlet 104. The dispensing tube 100 defines a channel 110 extending therethrough from the proximal end 102 to the distal end 106. An outlet 108 is disposed at the distal end 106 and is in fluid communication with the channel 110. The liquid material may move through the dispensing tube 100 and out through the outlet 108 along a dispensing axis A extending axially from the inlet 104 to the outlet 108.

The dispensing tube 100 may be manufactured such that the channel 110 has a constant cross-sectional shape and/or diameter, or it may have a variable shape or diameter. In some aspects, for example, the channel 110 may include multiple sections that have different dimensions. Referring to FIG. 5, for example, the channel 110 may include a first section 112 separated from a second section 114 by a transition point 116. The first section 112 of the channel 110 may have a first diameter D1, while the second section 114 may taper to the outlet 108 of the dispensing tube 100 having a second diameter D2. As depicted in the drawings, the first diameter D1 may be greater than the second diameter D2.

The first section 112 of the channel 110 may be defined between the proximal end 102 and the transition point 116 and may be substantially cylindrical and have a constant diameter therethrough. In some aspects, the inlet 104 may have the same cross-sectional shape as the first section 112 (e.g., circular) and may be dimensioned to have a diameter equal to the first diameter D1.

The second section 114 may be defined between the transition point 116 and the distal end 106 and may be defined such that the channel 110 in that section has variable dimensions. In some aspects, the second section 114 may include a taper such that the channel 110 becomes narrower in the direction from the transition point 116 toward the distal end 106.

The dispensing tube 100 may be designed to have different first and second diameters D1, D2 based on manufacturing preferences, liquid material to be dispensed, dimensions of the dispensed material on a substrate, or another factor. The first diameter D1 may be greater than the second diameter D2, such that as the liquid material is moved through the dispensing tube 100 from the proximal end 102 to the distal end 106, the liquid material moves in a direction from the larger first diameter D1 to the smaller second diameter D2. As such, the larger first diameter D1 causes less pressure to be required to move the liquid material through the dispensing tube 100.

The first diameter D1 may be any suitable dimension, such as between about 0.05 mm and about 5 mm, between about 0.2 mm and about 3 mm, between about 0.5 mm and about 1 mm, or another suitable range. In some aspects, the first diameter D1 may be approximately 0.7 mm. The second diameter D2 may also be any suitable dimension, such as between about 0.001 mm and about 1 mm, between about 0.005 mm and about 0.5 mm, between about 0.01 mm and 0.1 mm, between about 0.02 mm and about 0.08 mm, between about 0.03 mm and about 0.06 mm, or another suitable range. In some aspects, the second diameter D2 may be approximately 0.05 mm. In further aspects, the second diameter D2 could be approximately 0.035 mm. It will be understood that different aspects exist that can utilize any combination of the disclosed ranges above for first and second diameters D1, D2, and that in aspects where the first diameter D1 is greater than the second diameter D2, the selected dimension of the first diameter D1 will be greater than the selected dimension of the second diameter D2.

Although the figures depict the dispensing tube 100 having two sections, it will be understood that the dispensing tube 100 may have a different number of sections, such as three, four, five, or any other number of sections. Each section may have dimensions that are different from all other sections, or, alternatively, dimensions that match one or more of the other sections. In some aspects, the dispensing tube 100 may have a single section, such as a section similar to the first section 112 and having a constant diameter along its length, or such as a section similar to the second section 114 and having a taper that defines a variable diameter along its length.

The dispensing tube 100 may further include a third section (not shown) that may either have a constant diameter throughout or, alternatively, may be tapered at the same tapering angle α as the second section 114 or at a different tapering angle. The third section may be adjacent to the first section 112, the second section 114, or both sections.

In some aspects, the taper present in the second section 114 may be constant. Referring to FIG. 6, the second section 114 may be tapered at a tapering angle α being measured relative to the dispensing axis A. It will be understood that the tapering angle α may be selected depending on manufacturing preferences and capabilities, as well as on parameters of the dispensing tube 100, such as length, thickness, desired diameters (e.g. diameter D1 or diameter D2), desired application, liquid to be dispensed, or other factors that can be affected by the taper specifications.

In an alternate aspect, the taper of the second section 114 may vary along its length. For example, the tapering angle α measured from the dispensing axis A may increase or decrease along the length of the second section 114. This may define the interior of the dispensing tube 100 that is adjacent the second section 114 as convex or concave, depending on if the tapering angle α is increasing or decreasing (not shown). The tapering angle α may be, for example, between about 1° and about 60°, between about 3° and about 30°, or between about 5° and about 15°.

The dispensing tube 100 may be manufactured out of different suitable materials, for example metals, plastics, alloys, and/or ceramics. In some aspects, the dispensing tube 100 may include steel, such as stainless steel. In an alternate aspect, the dispensing tube 100 may include a ceramic, such as a black ceramic, such as black zirconia. In yet another aspect, the dispensing tube 100 may include zirconia toughened alumina. The dispensing tube 100 may be manufactured to have a uniform makeup throughout, or, alternatively, having a different distribution of materials at various portions of the dispensing tube 100. It will be understood that the material from which the dispensing tube 100 is composed should allow for easy sliding of the liquid material and should not adversely affect the flow of the liquid material.

The dispensing tube 100 may include a combination of materials, either as a mixture or with different portions of the dispensing tube 100 including different materials. For example, the distal end 106 may include a different material than the proximal end 102, such as a harder material to withstand stresses associated with dispensing activities. In some aspects, a coating may be applied to the interior of the dispensing tube 100 such that the liquid material inside the channel 110 contacts the coating (not shown). It will be understood that a coating may be applied to other portions of the dispensing tube 100 as well, for example, to the exterior surface. In some aspects, the coating may include polytetrafluoroethylene (PTFE) or another suitable material.

The dispensing tube 100 may be secured to or within the hub 12 of the dispensing tip 10 via any suitable retention method as disclosed throughout this application. In some aspects, an adhesive 20 may be used to connect the dispensing tube 100 to the hub 12. The adhesive 20 may include any suitable adhesive. It will be understood that the composition of the adhesive 20 will depend on the materials present in the dispensing tube 100 and the hub 12, such that the adhesive can effectively secure the components to one another. In some aspects, the adhesive 20 may include a flowable adhesive having a low enough viscosity to easily enter the space between the dispensing tube 100 and the hub 12. The adhesive 20 may then be allowed to solidify so as to become less viscous. The adhesive 20 may solidify by itself upon passage of a given drying period without external intervention, or, alternatively, the adhesive 20 may be treated to stimulate a curing process (e.g., exposure to ultraviolet light, high temperatures, a curing fluid, or another factor that accelerates the curing process).

Referring to FIGs 3 and 4, the dispensing tube 100 may be inserted partially into a receptacle 18 disposed at or adjacent the distal end 15 of the hub 12 on the dispensing tip 10. The receptacle 18 is in fluid communication with the chamber 19 and can receive the liquid material from the chamber 19 and allow it to pass through to the dispensing tube 100. The receptacle 18 may include one or more retention members 21 configured to engage the dispensing tube 100, the adhesive 20, or both, in order to improve engagement of the dispensing tube 100 with the rest of the dispensing tip 10. The retention members 21 may include ribs, notches, protrusions, threads, or other structural features configured to increase contact between the dispensing tube 100 and the hub 12. In some aspects, the dispensing tube 100 may have retention members (not shown) in addition or instead of the retention members 21 on the dispensing tip 10.

The dispensing tip 10 having the dispensing tube 100 may be disconnected from the dispensing apparatus with which they are used, for example the syringe 30, and a different dispensing tip 10 having a dispensing tube 100 may be connected instead. During the dispensing process, it may be advantageous to replace the dispensing tip 10, the dispensing tube 100, or the combination thereof due to sustained damage during operation. The components described throughout this application are subjected to various stresses during dispensing, and they may break, bend, crack, or otherwise sustain damage after a set period of dispensing time or a given amount of material dispensed. Components of the dispensing tip 10, such as the hub 12 or the dispensing tube 100, may become clogged with one or more materials over the course of operation. Additionally, it may be preferred to replace the components when the dispensing process is non-operational due to maintenance or other reasons. Furthermore, different aspects of the dispensing tip 10 and the dispensing tube 100 discussed herein may be utilized with specific liquid materials to be dispensed or with specific dispensing patterns, and when a different liquid material is to be introduced or a different dispensing pattern selected, the dispensing tip 10 and/or the dispensing tube 100 may be replaced.

Various dispensing parameters are preset or preprogrammed based on the desired dispensing patterns, quantities, durations, speeds, and other factors. The dispensing parameters are related to the structural features of the dispensing tip 10, specifically those related to the hub 12 and/or the dispensing tube 100. The pressure within the hub 12 and the dispensing tube 100, the flow rate, and the viscosity of the liquid material, as well as other factors, may correspond to unique dispensing parameters. In existing dispensing systems, dispensing tubes often had varying diameters, the dispensing tubes were prone to bends or kinks, and material often got stuck in the dispensing tube, creating obstacles for flow. Existing dispensing tubes have large manufacturing tolerances, which results in inconsistently sized outlet orifices and channels extending through the tube. This may result in unreliable and inconsistent dispensing of the material and in a different set of dispensing parameters. Based on the manufacturing process of the existing tubes and the materials used, the tolerances could not be lowered. When the damaged dispensing tubes are replaced, the new tubes likely have different properties due to different bends, kinks, and differently dimensioned inside diameters. This affects how the material is dispensed, and the dispensing parameters must be adjusted or re-calibrated to ensure consistent dispensing.

The disclosed aspects of the dispensing tubes 100 herein, on the other hand, have smaller inner diameter tolerances, and the exemplary shapes and dimensions of the dispensing tubes 100 provide increased durability and allow for more consistent dispensing of the material over the lifespan of the tube. Additionally, due to the smaller tolerances, there is greater consistency in dimensions among individual dispensing tubes 100. This improved consistency allows for less alteration of the dispensing parameters of the dispensing system and decreases the need to re-calibrate the dispensing parameters when tubes or tips are replaced.

The dispensing tubes 100 disclosed throughout this application may have smaller outlets 108 than corresponding outlet orifices in existing dispensing tubes. The liquid material may be dispensed from the dispensing tube 100 onto a substrate (not shown) in a variety of shapes and patterns, such as elongated strands and/or droplets. In aspects where droplets are formed on the substrate, the disclosed dispensing tubes 100 may allow for the dispensed droplets to be smaller than those dispensed from existing dispensing tubes, for example, about two-thirds of the size of existing droplets, about one half the size, about one-third the size, or about one-fourth the size of existing droplets. The smaller size of the deposited liquid material may be advantageous because it allows for better control of how much liquid material is dispensed and where on the substrate it is dispensed. This also provides option for better control of dispensing patterns, such as density of dispensed droplets, shape of the droplets, and duration of dispensing.

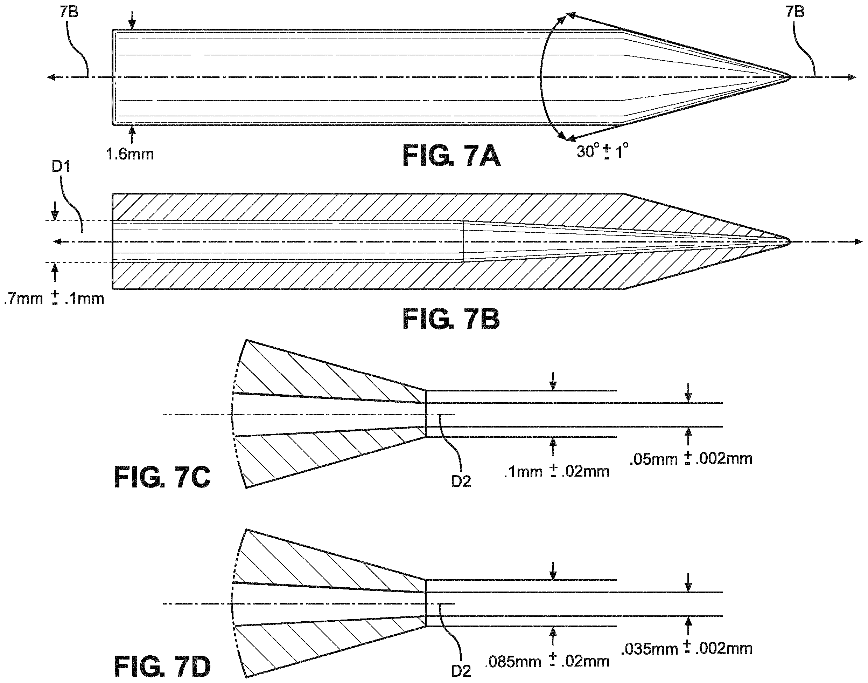

In dispensing tubes having a taper (e.g., having a first section 112 and a second section 114), such as about 30 degrees as shown in FIG. 7A, the flow of material is improved over existing dispensing tubes having a constant inner diameter from their respective inlets to the outlets. Currently used dispensing tubes often have an undesirably high resistance to flow of the liquid material therethrough. The existing dispensing tubes thus require application of high pressures to move the liquid material into, through, and out of the dispensing tube. This may result in faster degradation of the dispensing tube, wasteful energy used to apply the pressure, and inconsistent dispensing of the liquid material and formation of deposits on substrates. This also requires more time to achieve the desired deposit size. In the aspects of dispensing tubes 100 disclosed throughout this application, when the liquid material moves from the first section 112 having a first diameter D1 into the second section 114 having a second diameter D2 that is smaller than the first diameter D1, the velocity of the liquid material traveling through the second section 114 increases relative to its velocity in the first section 112. As such, less pressure may be required to move a desired quantity of the liquid material through the dispensing tube 100 than in existing dispensing tubes. For example, the pressure can range from about 5 psig to about 100 psig, and the velocity can range from about 0.001 m/sec to about 1.0 m/sec.

While systems and methods have been described in connection with the various embodiments of the various figures, it will be appreciated by those skilled in the art that changes could be made to the embodiments.

## Claims

1. A tube for use with a dispensing syringe for dispensing a liquid material, the tube comprising:
a body having a proximal end and a distal end opposite the proximal end, the proximal end being configured to be connected to a hub that is configured to be connected to the dispensing syringe;
a channel extending through the body between the proximal end and the distal end;
an inlet at the proximal end, the inlet being in fluid communication with the channel and configured to receive the liquid material into the channel; and
an outlet at the distal end, the outlet being in fluid communication with the channel,
wherein the channel is partially tapered in the distal direction such that the proximal end has a first inner diameter and the outlet has a second inner diameter that is smaller than the first inner diameter.

2. The tube of claim 1, wherein the second inner diameter is between about 5 µm and about 500 µm.

3. The tube of claim 2, wherein the second inner diameter is between about 30 µm and about 60 µm.

4. The tube of claim 3, wherein the second inner diameter is about 50 µm.

5. The tube of claim 3, wherein the second inner diameter is about 35 µm.

6. The tube of any preceding claim, wherein the channel includes a dispensing axis disposed centrally within the channel, and the tapered portion of the channel includes a tapering angle relative to the dispensing axis.

7. The tube of claim 6, wherein the tapering angle is between about 3° and about 30°.

8. The tube of claim 7, wherein the tapering angle is between about 5° and about 15°.

9. The tube of any preceding claim, wherein the tube is composed of a ceramic.

10. The tube of claim 9, wherein the ceramic is black ceramic.

11. A dispensing apparatus for dispensing a liquid material, the dispensing apparatus comprising:
a barrel having an interior compartment configured to receive the liquid material; and
a dispensing tip configured to be removably connected to the barrel, the dispensing tip having a chamber in fluid communication with the interior compartment of the barrel, the chamber being configured to receive the liquid material from the interior compartment; and
wherein the dispensing tip includes the dispensing tube of any preceding claim, the dispensing tube being configured to receive the liquid material into the channel at the inlet and to dispense the liquid material out of the outlet.

12. The dispensing apparatus of claim 11, further comprising a second dispensing tip that is configured to be removably connected to the barrel in place of the first dispensing tip.

13. A method of dispensing a liquid material, the method comprising:
providing the liquid material into a dispensing apparatus, the dispensing apparatus having a tube for dispensing the liquid material, the tube having a channel extending therethrough;
moving the liquid material through a first portion of the channel having a first inner diameter;
moving the liquid material through a second, tapered portion of the channel to an outlet having a second inner diameter, the second inner diameter being smaller than the first inner diameter; and
forming a deposit of the liquid material on a substrate.

14. The method of claim 13, further comprising:
removing the tube from the dispensing apparatus; and
connecting a second tube to the dispensing apparatus.
